# EUROPEAN PATENT APPLICATION

(11) **EP 1 672 060 A1**
(43) Date of publication of application: **21.06.2006**
(21) Application number: 04788321.0
(22) Date of filing: 29.09.2004
(51) Int. Cl.: C12N 9/22, C12N 15/55, C12P 19/34

(54) **POLYPEPTIDE HAVING RNASE III ACTIVITY**

(30) Priority: 30.09.2003 JP 2003342260; 08.12.2003 JP 2003409638
(71) Applicant: TAKARA BIO INC., Otsu-shi, Shiga 520-2193 (JP)
(72) Inventor: TOMONO, Jun, Okayama 710-0845 (JP); UENO, Harumi, c/o Takara Bio Inc., Otsu-shi, Shiga 520-2193 (JP); SAGAWA, Hiroaki, c/o Takara Bio Inc., Otsu-shi, Shiga 520-2193 (JP); KATO, Ikunoshin, c/o Takara Bio Inc., Otsu-shi, Shiga 520-2193 (JP)
(74) Representative: Schnappauf, Georg
(86) International application number: PCT/JP2004/014255
(87) International publication number: WO 2005/030948

(57) **Abstract**

A polypeptide having an RNase III activity with which the length of a dsRNA degradation product can be easily controlled depending on reaction conditions and, in preparing a dsRNA having a length allowing it to serve as an siRNA in RNA interference, a low-molecular weight product having little RNA interferring effect is scarcely formed; a method of degrading a dsRNA with the use of the above polypeptide; and a composition and a kit for the above method.

SEQUENCE LISTING

<110>  TAKARA BIO INC.

<120>  Polypeptide having RNaseIII activity

<130>  664746

<150>  JP 2003-342260
<151>  2003-09-30

<150>  JP 2003-409638
<151>  2003-12-08

<160>  17

<170>  PatentIn version 3.1

<210>  1
<211>  678
<212>  DNA
<213>  Shewanella sp.Ac10

<400>  1
atggaaccca ttaaaaattt gccgcgtttg tgccgtactt taggttatga gttcaataat      60

attgaattac ttattcaggc cttaacacat cgtagcgcag caaataaaca taatgagcgt     120

ttagagtttt taggtgattc gattttatcg atagccattt cagatgcctt atatcatcag     180

tttccaaagg cgactgaagg tgatttaagc cgaatgcgcg ccactttagt caaaggtgac     240

acgctgacaa tcatagctaa agagttcaag ctaggtgatt atttgtattt aggtcctggt     300

gaactcaaaa gtggtggctt tagacgcgaa tctattttag ctgatgctgt agaggctatt     360

attggtgctg tctatcttga tgctgatatt gaagtgtgcc gcaagctatt attatcatgg     420

tatcaagagc gtttagctga gattaaaccg ggtattaatc aaaaagatcc gaagacaata     480

ttgcaagaat acctgcaagg ttttaaaaag ccattgcctg attaccaagt tgttgcagta     540

gaaggtgaag cccatgatca aaccttcacc gtagaatgta aaattagtga attagataaa     600

gttgtcaccg gtgtggcaag ttcaagaaga aaagctgaac agcttgccgc tgctcaggta     660

ttggagctac tgaataaa                                                   678

<210>  2
<211>  39
<212>  DNA
<213>  Artificial

<220>
<223>  Synthetic primer 1 to amplify a gene encoding Shewanella sp.AC10 RNaseIII

<400>  2
cagattccac gaattcgatg gaacccatta aaaatttgc                             39

<210>  3
<211>  37
<212>  DNA
<213>  Artificial

<220>
<223>  Synthetic primer 2 to amplify a gene encoding Shewanella sp.AC10 RNaseIII

<400> 3
ggagaggtct ggatccttat ttattcagta gctcctt                                37

<210> 4
<211> 226
<212> PRT
<213> Shewanella sp. Ac10

<400> 4

Met Glu Pro Ile Lys Asn Leu Pro Arg Leu Cys Arg Thr Leu Gly Tyr
1               5                   10                  15

Glu Phe Asn Asn Ile Glu Leu Leu Ile Gln Ala Leu Thr His Arg Ser
            20                  25                  30

Ala Ala Asn Lys His Asn Glu Arg Leu Glu Phe Leu Gly Asp Ser Ile
        35                  40                  45

Leu Ser Ile Ala Ile Ser Asp Ala Leu Tyr His Gln Phe Pro Lys Ala
    50                  55                  60

Thr Glu Gly Asp Leu Ser Arg Met Arg Ala Thr Leu Val Lys Gly Asp
65                  70                  75                  80

Thr Leu Thr Ile Ile Ala Lys Glu Phe Lys Leu Gly Asp Tyr Leu Tyr
                85                  90                  95

Leu Gly Pro Gly Glu Leu Lys Ser Gly Gly Phe Arg Arg Glu Ser Ile
            100                 105                 110

Leu Ala Asp Ala Val Glu Ala Ile Ile Gly Ala Val Tyr Leu Asp Ala
        115                 120                 125

Asp Ile Glu Val Cys Arg Lys Leu Leu Leu Ser Trp Tyr Gln Glu Arg
    130                 135                 140

Leu Ala Glu Ile Lys Pro Gly Ile Asn Gln Lys Asp Pro Lys Thr Ile
145                 150                 155                 160

Leu Gln Glu Tyr Leu Gln Gly Phe Lys Lys Pro Leu Pro Asp Tyr Gln
                165                 170                 175

Val Val Ala Val Glu Gly Glu Ala His Asp Gln Thr Phe Thr Val Glu
            180                 185                 190

Cys Lys Ile Ser Glu Leu Asp Lys Val Val Thr Gly Val Ala Ser Ser
        195                 200                 205

Arg Arg Lys Ala Glu Gln Leu Ala Ala Ala Gln Val Leu Glu Leu Leu
    210                 215                 220

Asn Lys
225

<210> 5
<211> 243
<212> PRT
<213> Artificial

<220>
<223> An expression peptide sequence of Shewanella sp.AC10 RNaseIII

<400> 5

Met Asn His Lys Val His His His His His His Ile Glu Gly Arg Asn
1               5                   10                  15

Ser Met Glu Pro Ile Lys Asn Leu Pro Arg Leu Cys Arg Thr Leu Gly
            20                  25                  30

Tyr Glu Phe Asn Asn Ile Glu Leu Leu Ile Gln Ala Leu Thr His Arg
        35                  40                  45

Ser Ala Ala Asn Lys His Asn Glu Arg Leu Glu Phe Leu Gly Asp Ser
    50                  55                  60

Ile Leu Ser Ile Ala Ile Ser Asp Ala Leu Tyr His Gln Phe Pro Lys
65                  70                  75                  80

Ala Thr Glu Gly Asp Leu Ser Arg Met Arg Ala Thr Leu Val Lys Gly
                85                  90                  95

Asp Thr Leu Thr Ile Ile Ala Lys Glu Phe Lys Leu Gly Asp Tyr Leu
            100                 105                 110

Tyr Leu Gly Pro Gly Glu Leu Lys Ser Gly Gly Phe Arg Arg Glu Ser
        115                 120                 125

Ile Leu Ala Asp Ala Val Glu Ala Ile Ile Gly Ala Val Tyr Leu Asp
    130                 135                 140

Ala Asp Ile Glu Val Cys Arg Lys Leu Leu Leu Ser Trp Tyr Gln Glu
145                 150                 155                 160

Arg Leu Ala Glu Ile Lys Pro Gly Ile Asn Gln Lys Asp Pro Lys Thr
                165                 170                 175

Ile Leu Gln Glu Tyr Leu Gln Gly Phe Lys Lys Pro Leu Pro Asp Tyr
            180                 185                 190

Gln Val Val Ala Val Glu Gly Glu Ala His Asp Gln Thr Phe Thr Val
        195                 200                 205

Glu Cys Lys Ile Ser Glu Leu Asp Lys Val Val Thr Gly Val Ala Ser
    210                 215                 220

Ser Arg Arg Lys Ala Glu Gln Leu Ala Ala Ala Gln Val Leu Glu Leu

225                 230                 235                 240

Leu Asn Lys

<210>  6
<211>  720
<212>  DNA
<213>  Artificial

<220>
<223>  red-shifted green fluorescence protein

<400>  6
atggctagca aaggagaaga actcttcact ggagttgtcc caattcttgt tgaattagat      60

ggtgatgtta acggccacaa gttctctgtc agtggagagg gtgaaggtga tgcaacatac     120

ggaaaactta ccctgaagtt catctgcact actggcaaac tgcctgttcc atggccaaca     180

ctagtcacta ctctgtgcta tggtgttcaa tgcttttcaa gatacccgga tcatatgaaa     240

cggcatgact ttttcaagag tgccatgccc gaaggttatg tacaggaaag gaccatcttc     300

ttcaaagatg acggcaacta caagacacgt gctgaagtca agtttgaagg tgataccctt     360

gttaatagaa tcgagttaaa aggtattgac ttcaaggaag atggaaacat tctgggacac     420

aaattggaat acaactataa ctcacacaat gtatacatca tggcagacaa acaaaagaat     480

ggaatcaaag tgaacttcaa gacccgccac aacattgaag atggaagcgt tcaactagca     540

gaccattatc aacaaaatac tccaattggc gatggccctg tccttttacc agacaaccat     600

tacctgtcca cacaatctgc cctttcgaaa gatcccaacg aaaagagaga ccacatggtc     660

cttcttgagt ttgtaacagc tgctgggatt acacatggca tggatgaact gtacaactga     720

<210>  7
<211>  42
<212>  DNA
<213>  Artificial

<220>
<223>  Synthetic primer dsr-1 to amplify a gene encoding red-shifted green fluorescence protein

<400>  7
gggtaatacg actcactata gggagaatgg ctagcaaagg ag                        42

<210>  8
<211>  42
<212>  DNA
<213>  Artificial

<220>
<223>  Synthetic primer dsr-2 to amplify a gene encoding red-shifted green fluorescence protein

<400>  8
gggtaatacg actcactata gggagatcag ttgtacagtt ca                        42

<210>  9
<211>  42
<212>  DNA
<213>  Artificial

<220>
<223>  Synthetic primer dsl-1 to amplify a gene encoding luciferase

<400>  9
gggtaatacg actcactata gggagaatgg aagacgccaa aa                    42


<210>  10
<211>  42
<212>  DNA
<213>  Artificial

<220>
<223>  Synthetic primer dsl-2 to amplify a gene encoding luciferase

<400>  10
gggtaatacg actcactata gggagagaac gtgtacatcg ac                    42


<210>  11
<211>  42
<212>  DNA
<213>  Artificial

<220>
<223>  Synthetic primer dsl-3 to amplify a gene encoding luciferase

<400>  11
gggtaatacg actcactata gggagaggca gatggaacct ct                    42


<210>  12
<211>  66
<212>  PRT
<213>  Thermotoga maritima

<400>  12

Met Arg Gly Lys Val Lys Trp Phe Asp Ser Lys Lys Gly Tyr Gly Phe
1               5                   10                  15


Ile Thr Lys Asp Glu Gly Gly Asp Val Phe Val His Trp Ser Ala Ile
            20                  25                  30


Glu Met Glu Gly Phe Lys Thr Leu Lys Glu Gly Gln Val Val Glu Phe
        35                  40                  45


Glu Ile Gln Glu Gly Lys Lys Gly Pro Gln Ala Ala His Val Lys Val
    50                  55                  60


Val Glu
65


<210>  13
<211>  198
<212>  DNA
<213>  Thermotoga maritima

<400>  13
atgagaggaa aggttaagtg gttcgattcc aagaagggct acggattcat cacaaaggac      60

gaaggaggag acgtgttcgt acactggtca gccatcgaaa tggaaggttt caaaactctg     120

aaggaaggcc aggtcgtcga gttcgagatt caggaaggca agaaaggtcc acaggcagcg     180

cacgtgaaag tagttgag                                                   198

<210>  14
<211>  20
<212>  DNA
<213>  Artificial

<220>
<223>  Synthetic primer rsGFP-F to amplify a gene encoding rsGFP

<400>  14
gccacaacat tgaagatgga                                                  20


<210>  15
<211>  20
<212>  DNA
<213>  Artificial

<220>
<223>  Synthetic primer rsGFP-R to amplify a gene encoding rsGFP

<400>  15
gaaagggcag attgtgtgga                                                  20

<210>  16
<211>  20
<212>  DNA
<213>  Artificial

<220>
<223>  Synthetic primer Neo-F to amplify a gene encoding Neo

<400>  16
atagcgttgg ctacccgtga                                                  20


<210>  17
<211>  20
<212>  DNA
<213>  Artificial

<220>
<223>  Synthetic primer Neo-R to amplify a gene encoding Neo

<400>  17
gaaggcgata gaaggcgatg                                                  20

## Description

### Technical Field

The present invention relates to a newly isolated polynucleotide and a polypeptide having RNase III encoded by the polynucleotide as well as use and production of the polynucleotide and the polypeptide.

### Background Art

An RNase III is an enzyme that is capable of acting specifically on a double-stranded RNA (dsRNA) to produce an oligonucleotide of 15 nucleotides on average having a phosphate group at the 5' terminus. For example, this enzyme exists in an *Escherichia coli* 30S extract. Also, it has been reported that similar enzymes exist in bovine thymus and chicken embryo. In most case, the enzyme has been used in a method for distinguishing a secondary structure of RNA-RNA from a single-stranded RNA or the like (see, for example, Non-patent Document 1).

Recently, a technique called RNA interference has been reported. This technique is based on a phenomenon in which an mRNA is degraded with a dsRNA in a sequence-specific manner, resulting in suppression of gene expression. The beginning of the finding that a dsRNA can be used for gene silencing was a study in which an antisense was used in *Caenorhabditis elegans.* In 1995, Guo and Kemphues carried out an experiment to suppress a gene called par-1 using an antisense RNA. When an antisense RNA was added, the expression of par-1 was suppressed as expected. Surprisingly, a sense RNA which was used as a control also suppressed the expression of par-1, resulting in a phenotype of a par-1 mutant (see, for example, Non-patent Document 2).
This contradiction was resolved by Fire et al. in 1998. When an antisense RNA or a sense RNA is synthesized using an RNA polymerase, a small amount of an inverse RNA is unintentionally produced in a nonspecific manner. It has been shown that a dsRNA formed due to the contamination is the essential entity for the gene silencing, an antisense RNA or a sense RNA cannot suppress gene expression, and a dsRNA formed by annealing an antisense RNA to a sense RNA can efficiently suppress gene expression (see, for example, Non-patent Document 3, Patent Document 1).

In RNA interference, an enzyme called a Dicer produces a small RNA (a short interfering RNA (siRNA)) from a dsRNA (see, for example, Non-patent Document 4, Patent Document 2).
It is considered that the siRNA produced as a result of the action of the enzyme is incorporated into a complex called an RNA induced silencing complex (RISC), and the complex recognizes and degrades a target mRNA. However, the exact functions of the respective factors supposed to be involved in RNA interference have been unknown in many cases (see, for example, Non-patent Document 5).

An RNase III derived from a microorganism is also utilized for preparation of an siRNA (see Non-patent Document 6). For example, an RNase III from *Escherichia coli* is commercially available from Epicentre or Ambion as such an RNase III and is used for preparation of an siRNA. The reactivity of the RNase III from *Escherichia coli* is high. For example, even if a long dsRNA of 500 base pairs or more is used as a template, it is immediately degraded into a small molecule of about 10 base pairs. In this case, partial degradation of the long dsRNA must be conducted by modifying the conditions (for example, by lowering the reaction temperature, or by shortening the reaction time) in order to prepare an siRNA of about 21 base pairs which is considered to be useful for RNA interference. However, since the reactivity of the RNase III from *Escherichia coli* is high as described above, the enzyme has drawbacks that it is difficult to control the reaction conditions, and a small molecule tends to be produced even under conditions for partial degradation.
As described above, an RNase III for which reaction conditions can be readily controlled upon preparation, from a double-stranded RNA, of an siRNA of a length with which an effect in RNA interference can be expected, and which does not tend to produce a small molecule whose RNA interference effect is low has been desired.

Patent Document 1: US 6506559
Patent Document 2: WO 01/68836
Non-patent Document 1: Enzyme Nomenclature (http://www.chem.qmul.ac.uk/iubmb/enzyme/), EC 3.1.26.3
Non-patent Document 2: Guo, S. et al., Cell, 1995, vol.81, p.611-620
Non-patent Document 3: Fire, A. et al., Nature, 1998, vol.39, p.806-811
Non-patent Document 4: Bernstein, E. et al., Nature, 2001, vol.409, p.363-366
Non-patent Document 5: Tabara, H. et al., Cell, 2002, vol.109, p.861-871
Non-patent Document 6: Zhang, H. et al., The EMBO Journal, 2002, vol.21, No.21, p.5875-5885

### Disclosure of Invention

### Problems to be Solved by the Invention

The main object of the present invention is to provide a novel polypeptide having an RNase III activity, with which a length of a dsRNA degradation product can be readily controlled by reaction conditions, and which does not tend to produce a small molecule whose RNA interference effect is low upon preparation of a dsRNA of a length that is capable of functioning in RNA interference as an siRNA. It is also an object of the present invention to provide an efficient method for preparing an siRNA using the polypeptide, which is different from conventional ones.

### Means to Solve the Problems

For easy control of reaction conditions, the present inventors have intensively examined a polypeptide having an RNase III activity that can be heat-inactivated at a temperature lower than an RNase III derived from a mesophile, and with which mild degradation conditions can be set utilizing the thermosensitivity. As a result, the present inventors have found that a polypeptide having an RNase III activity derived from a cold-adapted microorganism has an RNase III activity with which a length of a dsRNA degradation product can be readily controlled by reaction conditions, and which does not tend to produce a small molecule whose RNA interference effect is low upon preparation of an siRNA of a length that is capable of functioning in RNA interference as an siRNA. The present inventors have attempted to clone a polynucleotide encoding a polypeptide having an RNase III activity from a cold-adapted microorganism *Shewanella* sp. Ac10 which can grow at 4°C, successfully expressed the polypeptide having an RNase III activity of interest, and found that the activity of the RNase III is preferable for preparation of an siRNA. Thus, the present invention has been completed.

The first aspect of the present invention relates to a polypeptide having an RNase III activity, which is derived from a microorganism, and with which a dsRNA degradation product of a length within a specific range that is effective for RNA interference can be obtained after complete degradation.

The second aspect of the present invention relates to a polypeptide having an RNase III activity, for which reaction conditions can be readily controlled, and with which a dsRNA degradation product of a length within a specific range larger than a final degradation product obtained by treating a dsRNA with an RNase III from *Escherichia coli* can be obtained.

The third aspect of the present invention relates to a polypeptide having an RNase III activity, of which the dsRNA degradation velocity is slower than the dsRNA degradation velocity of an RNase III from *Escherichia coli,* and for which reaction conditions can be readily controlled.

The fourth aspect of the present invention relates to a polypeptide having an RNase III activity, of which the dsRNA degradation velocity is slower than the dsRNA degradation velocity of an RNase III from *Escherichia coli*, for which reaction conditions can be readily controlled, and which does not tend to produce a small dsRNA degradation product of about 10 base pairs.

According to the first to fourth aspects, the polypeptide is preferably derived from a cold-adapted microorganism. Although it is not intended to limit the present invention, for example, it is preferably a microorganism of the genus *Shewanella.*

The fifth aspect of the present invention relates to a polypeptide having an RNase III activity, with which a dsRNA degradation product of a length within a specific range larger than a final degradation product obtained by treating a dsRNA with an RNase III from *Escherichia coli* can be obtained at will depending on the reaction conditions, and which contains an amino acid sequence selected from the group consisting of:
(a) the amino acid sequence of SEQ ID NO:4;
(b) an amino acid sequence in which one or several amino acid(s) is(are) substituted, deleted, inserted or added in the amino acid sequence of SEQ ID NO:4; and
(c) an amino acid sequence encoded by a nucleotide sequence that is capable of hybridizing to the nucleotide sequence of SEQ ID NO:1 under stringent conditions.

The sixth aspect of the present invention relates to the polypeptide of the first to fourth aspects, which is a fusion protein with a protein having an activity of binding to a nucleic acid.

The seventh aspect of the present invention relates to a method for degrading a dsRNA, using the polypeptide of the first to sixth aspects.

According to the seventh aspect, although it is not intended to limit the present invention, for example, the dsRNA degradation product is a dsRNA that is capable of functioning in RNA interference as an siRNA. The method of the present invention may be conducted in the presence of a protein having an activity of binding to a nucleic acid. The polypeptide used may be a fusion protein of a protein having an activity of binding to a nucleic acid and the polypeptide of the first to fourth aspects. Although it is not intended to limit the present invention, for example, the protein having an activity of binding to a nucleic acid is a cold shock protein derived from a thermophilic bacterium or a thermostable bacterium. Although it is not intended to limit the present invention, for example, the cold shock protein is cold shock protein B from *Thermotoga maritima.*

The eighth aspect of the present invention relates to a composition for degrading a dsRNA, which is used for the method of the seventh aspect, and which contains the polypeptide of the first to sixth aspects.

The ninth aspect of the present invention relates to a kit for degrading a dsRNA, which is used for the method of the seventh aspect, and which contains the polypeptide of the first to sixth aspects.

The tenth aspect of the present invention relates to a nucleic acid which has a nucleotide sequence selected from the group consisting of:
(a) the nucleotide sequence of SEQ ID NO:1;
(b) a nucleotide sequence in which one or several nucleotide(s) is(are) substituted, deleted, inserted or added in the nucleotide sequence of SEQ ID NO:1; and
(c) a nucleotide sequence that is capable of hybridizing to the nucleotide sequence of SEQ ID NO:1 under stringent conditions.

The eleventh aspect of the present invention relates to a method for producing a polypeptide having an RNase III activity using the nucleic acid of the tenth aspect.

### Effects of the Invention

The present invention provides a polypeptide having an RNase III activity, which is derived from a cold-adapted microorganism, and for which reaction conditions can be readily controlled. Furthermore, a convenient and efficient method for producing a dsRNA of a length that is capable of functioning in RNA interference as an siRNA is provided using the polypeptide of the present invention.

### Best Mode for Carrying Out the Invention

As used herein, a polypeptide having an RNase III activity refers to an endonuclease that degrades a double-stranded RNA (dsRNA). Generally, it completely digests a dsRNA to produce a small dsRNA fragment of about 10 base pairs. The polypeptide can degrade a dsRNA (either naturally occurring or artificially modified) as a substrate. Although it is not intended to limit the present invention, for example, a dsRNA substituted with fluorine-CMP w2'-fluorine-UMP may be used.

As used herein, a dsRNA refers to an RNA forming a double-stranded structure. Although it is not intended to limit the present invention, for example, it refers to an RNA forming a double-stranded structure consisting of an mRNA to be subjected to RNA interference and an RNA having a nucleotide sequence complementary to the mRNA.
The dsRNAs include RNA degradation products retaining double-stranded structures including short interfering RNAs (siRNAs) which are useful for RNA interference. The dsRNA or a degradation product thereof may have a single-stranded portion on the 3' and/or 5' side(s).

As used herein, a dsRNA that is capable of functioning in RNA interference as an siRNA refers to, for example, a dsRNA of a specific length within a range of about 10 to 100 base pairs although it is not intended to limit the present invention. Furthermore, for example, an siRNA of a length that is capable of functioning in RNA interference as an siRNA may be a dsRNA of a length within a range of about 15 to 30 base pairs, particularly 20 to 25 base pairs.

As used herein, a dsRNA of a length within a specific range refers to a dsRNA that is capable of functioning in RNA interference as an siRNA which is characterized by a low abundance of a small dsRNA degradation product of about 10 base pairs. Although it is not intended to limit the present invention, it may be a dsRNA of a length within a range of about 10 to 100 base pairs, preferably about 15 to 30 base pairs, more preferably 20 to 25 base pairs.

As used herein, one for which reaction conditions can be readily controlled refers to, for example in case of a dsRNA degradation reaction, one for which the conditions for partial degradation can be readily determined using the resulting small product as an index (using the length of the dsRNA degradation product as an index) because of the slow reaction velocity in the degradation reaction, although it is not intended to limit the present invention. That is, one that does not tend to produce a small product is preferable. Although it is not intended to limit the present invention, for example, one derived from a cold-adapted microorganism as described below can be preferably used as an enzyme for which reaction conditions can be readily controlled.

As used herein, complete degradation means a degradation reaction with a sufficient amount of an enzyme after passing a time sufficient for the amount of degradation product produced as a result of progress of the substrate degradation reaction to reach a stationary phase (an equilibrium state without change in amounts of the substrate and the degradation product). Partial degradation means degradation before reaching the complete degradation.

Microorganisms are generally classified into thermophilic microorganisms, mesophilic microorganisms and cold-adapted microorganisms according to the ranges of temperatures at which they can grow. Microorganisms that can grow at low temperatures are further subdivided into psychrotrophic microorganisms and psychrophilic microorganisms according to the optimal growth temperatures and the uppermost temperatures above which they can no longer grow. A microorganism having an uppermost growth temperature of 20°C or below and an optimal growth temperature of 15°C or below is generally called a psychrophilic microorganism, whereas a microorganism having an uppermost growth temperature of 20°C or above is generally called a psychrotrophic microorganism. As used herein, a cold-adapted microorganism refers to the above-mentioned cold-adapted microorganism. There is no specific limitation concerning the genus, the species or the strain of the microorganism that can be used as a source of a gene according to the present invention. For example, a cold-adapted microorganism classified into the genus *Shewanella* can be used. Such a microorganism can be readily obtained from a public microorganism depositary. Examples of microorganisms of the genus *Shewanella* include *Shewanella putrefaciens* SCRC-2874 (FERM BP-1625) and *Shewanella* sp. Ac10. However, it is needless to say that various cold-adapted microorganisms can be similarly used as sources of genes as described above.

Hereinafter, the present invention will be described in detail.
(1) The polypeptide having an RNase III activity of the present invention and the polynucleotide encoding the polypeptide
The polypeptide having an RNase III activity of the present invention is a polypeptide for which reaction conditions can be readily controlled. Although it is not intended to limit the present invention, an example thereof is a polypeptide having an RNase III activity for which reaction conditions can be readily controlled and with which a dsRNA degradation product of a length within a specific range larger than a final degradation product obtained by treating a dsRNA with an RNase III from *Escherichia coli* can be obtained.
The polypeptide of the present invention may be a polypeptide of which the dsRNA degradation velocity is slower than the dsRNA degradation velocity of an RNase III from *Escherichia coli,* for which reaction conditions can be readily controlled, and which does not tend to produce a small dsRNA degradation product of about 10 base pairs.
Thus, the polypeptide of the present invention can be used to readily obtain a dsRNA of a length within a specific range which has been difficult to obtain using a conventional RNase III from *Escherichia coli* by setting reaction conditions (temperature, time, enzyme concentration, etc.) at will. Furthermore, it is preferably a polypeptide with which a dsRNA of a length within a specific range can be readily obtained without adding a special reagent such as a manganese ion to the enzyme buffer.

A polypeptide having an RNase III activity of which the dsRNA degradation velocity is slower than the dsRNA degradation velocity of an RNase III from *Escherichia coli*, and for which reaction conditions can be readily controlled exemplifies another embodiment of the polypeptide of the present invention.
Although it is not intended to limit the present invention, for example, one of which the dsRNA degradation velocity is slower than the dsRNA degradation velocity of an RNase III from *Escherichia coli,* and which does not tend to produce a fragment of about 10 base pairs at the reaction velocity even after reacting for one hour is preferable according to the present invention.
Since the dsRNA degradation velocity of the polypeptide having an RNase III activity of the present invention is slower than the dsRNA degradation velocity of an RNase III from *Escherichia coli,* a dsRNA of an arbitrary specific length can be prepared by appropriately selecting the reaction time, the reaction temperature or the like. Partial degradation of a dsRNA which has been difficult using a conventional RNase III from *Escherichia coli* can be conducted by utilizing this property. Furthermore, the polypeptide having an RNase III activity of the present invention is a polypeptide which does not tend to produce a small dsRNA which hardly functions in RNA interference as an siRNA as compared with the RNase III from *Escherichia coli*. In other words, it is possible to conveniently and efficiently prepare a dsRNA that is capable of functioning in RNA interference as an siRNA using the polypeptide because the velocity of degrading a long dsRNA (about 500-1000 base pairs) of the polypeptide is slow as compared with an RNase III from *Escherichia coli,* and the polypeptide does not tend to produce a small degradation product of about 10 base pairs.

The polypeptide of the present invention can be used to prepare a dsRNA that is capable of functioning in RNA interference as an siRNA. Although it is not intended to limit the present invention, an example thereof is one having the amino acid sequence of SEQ ID NO:4. The polynucleotide encoding the polypeptide having an RNase III activity of the present invention is exemplified by one having the nucleotide sequence of SEQ ID NO:1. The polypeptides having an RNase III activity of the present invention, or the polynucleotides encoding the same, include one in which one or several amino acid(s), or nucleotide(s), is(are) substituted, deleted, inserted or added in the amino acid sequence, or the nucleotide sequence, as long as it has the above-mentioned function. Although it is not intended to limit the present invention, examples thereof include a polypeptide having the amino acid sequence of SEQ ID NO:5.
Furthermore, a polypeptide coded for by an amino acid sequence having a homology of at least 70%, preferably 80%, more preferably 90%, still more preferably 95% to the amino acid sequence disclosed by the present application (SEQ ID NO:4) is within the scope of the present invention as long as it has an RNase III activity.

Furthermore, a polynucleotide that hybridizes to the polynucleotide of SEQ ID NO:1 under stringent conditions and encodes a polypeptide having a dsRNA degradation activity equivalent to a polypeptide encoded by the polynucleotide of SEQ ID NO:1 is included. Examples of the "stringent hybridization conditions" include, but are not limited to, the conditions as described in a literature such as Sambrook et al., Molecular cloning, A laboratory manual 3^{rd} edition, 2001, Cold Spring Harbor Laboratory Press, e.g., incubation at a temperature of ["Tm of the probe to be used" - 25°C] overnight in a solution containing 6 x SSC (1 x SSC: 0.15 M NaCl, 0.015 M sodium citrate, pH 7.0), 0.5% SDS, 5 x Denhardt's (0.1% bovine serum albumin (BSA), 0.1% polyvinylpyrrolidone, 0.1% Ficoll 400) and 100 µg/ml salmon sperm DNA.

The present invention also encompasses a nucleic acid molecule that hybridizes to the polynucleotide of the present invention under less stringent hybridization conditions. Hybridization stringency and signal detection are changed mainly by manipulating the formamide concentration (a lower percentage of formamide results in reduced stringency), the salt concentration or the temperature. Examples of less stringent conditions include incubation at 37°C overnight in a solution containing 6 x SSPE (20 x SSPE = 3 M NaCl; 0.2 M NaH₂PO₄; 0.02 M EDTA, pH 7.4), 0.5% SDS, 30% formamide and 100 µg/ml salmon sperm blocking DNA followed by washing at 50°C using 1 x SSPE, 0.1% SDS. In order to accomplish further lower stringency, it is possible to conduct, following stringent hybridization, washing at a higher salt concentration (e.g., 5 x SSC).

The above-mentioned conditions can be modified by addition of and/or substitution with an alternative blocking reagent used for suppressing background in hybridization experiments. Representative blocking reagents include the Denhardt's reagent, BLOTTO, heparin, denatured salmon sperm DNA and commercially available formulations. In some case, it may be necessary to modify other factors of the hybridization conditions depending on the modification.

Furthermore, a nucleic acid having a homology of at least 60%, preferably 70%, more preferably 80%, still more preferably 90% to the nucleotide sequence disclosed by the present application (SEQ ID NO:1) is within the scope of the present invention.

The homology can be determined, for example, using a computer program DNASIS-Mac (Takara Bio), a computer algorithm FASTA (version 3.0; Pearson, W.R. et al., Pro. Natl. Acad. Sci., 85:2444-2448, 1988) or a computer algorithm BLAST (version 2.0; Altschul et al., Nucleic Acids Res. 25:3389-3402, 1997).

The polynucleotide of the present invention may be composed of any polyribonucleotide or polydeoxyribonucleotide, and may be an unmodified RNA or an unmodified DNA, or a modified RNA or a modified DNA. For example, a polynucleotide may be composed of a single-stranded or double-stranded DNA; a DNA that is a mixture of a single-stranded region and a double-stranded region; a single-stranded or double-stranded RNA; an RNA that is a mixture of single-stranded region and a double-stranded region; or a hybrid molecule containing a DNA and an RNA that may be single-stranded, more typically double-stranded or a mixture of a single-stranded region and a double-stranded region. The polynucleotide may contain one or more nucleotide(s), or DNA or RNA backbone(s), modified for its stability or for another reason. Examples of "modified" nucleotides include tritylated nucleotides and unusual nucleotides such as inosine. Various modifications can be conducted for DNAs and RNAs. Thus, "polynucleotides" include chemically, enzymatically or metabolically modified forms.

A method for producing the enzyme or the polypeptide using genetic engineering techniques is exemplified by a method in which a host cell is cultured under conditions under which an RNase III can be expressed, and the RNase III is collected from the culture. The host cell may be transformed with a plasmid in which a DNA sequence encoding the RNase III is inserted into a vector that replicates in the host organism along with suitable promoter, operator and terminator sequences having functions of expressing the enzyme in the host organism. Alternatively, the host cell may be transformed by integrating a DNA sequence encoding the protease into a DNA of the host cell along with suitable promoter, operator and terminator sequences having functions of expressing the enzyme in the host organism.

The RNase III of the present invention may be produced using genetic engineering techniques in which a conventional RNase III-encoding DNA is modified on the basis of the knowledge about the amino acid sequence of an RNase III obtained based on the polynucleotide encoding the RNase III or the like.
Although it is not intended to limit the present invention, the polypeptides having an RNase III activity of the present invention include ones having the following being added to the protein: a sequence derived from an expression vector such as an expression or translation enhancing sequence (e.g., Perfect DB sequence), or an amino acid sequence such as a tag sequence for purification of an expressed protein (e.g., His tag sequence) or a sequence for removing an N-terminal additional sequence of an expressed protein (e.g., Factor Xa sequence). Examples of such polypeptides include, but are not limited to, a polypeptide having an RNase III activity that has the amino acid sequence of SEQ ID NO:5.
Furthermore, the polypeptide having an RNase III activity of the present invention may be in a form of a fusion protein with a protein having an activity of binding to a nucleic acid (for example, a protein having an activity of synthesizing an RNA).

There is no specific limitation concerning a vector used for producing the polypeptide having an RNase III activity of the present invention. Any commercially available vector or expression system may be used. In particular, the pET system (Novagen) can be used, for example, although it is not intended to limit the present invention. In addition, a vector having a promoter that is capable of functioning at a low temperature can be preferably used. Examples thereof include the pCold-series vectors as described in WO 99/27117.
A method of production using the pCold-series vector as described in WO 99/27117 exemplifies one embodiment of the production method of the present invention.
Any vector can be preferably used according to the production method of the present invention as long as the vector can be used to express a polypeptide that can retain a function by which an siRNA can be produced.
Furthermore, a vector that is capable of expressing a polypeptide that is in a form of inclusion body upon expression of the polypeptide, but whose function can be restored by a subsequent refolding procedure may be included, provided that it is possible to obtain a polypeptide that can finally retain the function by which an siRNA can be produced.

The RNase III of the present invention may be collected according to a common means of collecting an *Escherichia coli* recombinant enzyme. Specifically, after an *Escherichia coli* recombinant is cultured, the cells can be separated from the culture using a conventional means of separation such as centrifugation or filtration. The cells are disrupted to prepare a cell-free extract which is used as a crude enzyme solution in the subsequent purification procedure. If the enzyme is secreted outside the cell, a culture supernatant from which the cells have been removed may be used as a crude enzyme solution. Although the crude enzyme solution may be used as it is, it can be used after recovery using a means such as ultrafiltration or precipitation, and powderization using an appropriate method, if required. A combination of common means of purifying an enzyme such as chromatography using as an appropriate cation exchange resin, anion exchange resin or hydroxyapatite, affinity chromatography, hydrophobic chromatography, gel filtration and the like can be used for purification.

If a polypeptide is to be produced using genetic engineering techniques or the product is to be used, the organism from which the polypeptide is derived greatly influences the efficiency of production or the effect upon use. Then, the polypeptide of the present invention can be produced in large quantities at low cost as compared with human Dicer. Furthermore, it is suitable for treatment of a large amount of a substrate because the amount of the substrate that remains uncleaved is little and the reaction time is short as compared with the Dicer.

### (2) The method for degrading a dsRNA using the polypeptide of the present invention

The method for degrading a dsRNA of the present invention is characterized by the use of the polypeptide as described in (1) above. For example, an siRNA of a length within a specific range can be prepared using the above-mentioned polypeptide by controlling reaction conditions (time, temperature, enzyme concentration, etc.) according to the method of the present invention. Although it is not intended to limit the present invention, for example, since a reaction can be conducted under mild reaction conditions as compared with those for the *Escherichia coli* RNase III (reaction temperature of 30°C), it is possible to conveniently and efficiently prepare an siRNA of a length with which an effect in RNA interference can be expected.

Furthermore, the method of the present invention is characterized in that it is carried out in the presence of a protein having an activity of binding to a nucleic acid. There is no specific limitation concerning the protein having an activity of binding to a nucleic acid as long as it results in promotion of a dsRNA degradation activity. For example, the above-mentioned protein having an activity of binding to an RNA can be preferably used.
Examples of the proteins having an activity of binding to an RNA include, but are not limited to, a cold shock protein (Csp). In particular, a cold shock protein that can function in a normal temperature range can be preferably used. A cold shock protein derived from a thermophilic bacterium or a thermostable bacterium is preferable. Although it is not intended to limit the present invention, for example, cold shock protein B (CspB) derived from *Thermotoga maritima* having the amino acid sequence of SEQ ID NO:12 (an amino acid sequence encoded by the nucleotide sequence of SEQ ID NO:13) as described in Protein Science, 8:394-403 (1999), the CspB protein derived from *Escherichia coli* as described in Mol. Microbiol., 11(5):833-839 (1994), or the CspB protein derived from *Bacillus subtilis* as described in J. Bacteriol., 174(20):6326-6335 (1992) can be preferably used.
It is possible to promoter an activity of degrading a dsRNA by using the CspB protein and the polypeptide as described in (1) above in combination. According to the method of the present invention, the protein having an activity of binding to a nucleic acid (e.g., a protein having an activity of synthesizing an RNA) may be in a form of a fusion protein with the polypeptide having an RNase III activity of the present invention.

### (3) Composition used for the method of the present invention

The composition of the present invention is a composition for efficiently conducting a reaction of degradation into a dsRNA of a specific length. For example, it is a composition for efficiently preparing an siRNA of a length with which an effect in RNA interference can be expected. The composition is a composition containing the polypeptide having an RNase III activity as described in (1) above.
The polypeptide having an RNase III activity may be a polypeptide having the following being added to the protein: a sequence derived from an expression vector such as an expression or translation enhancing sequence (e.g., Perfect DB sequence), or an amino acid sequence such as a tag sequence for purification of an expressed protein (e.g., His tag sequence) or a sequence for removing an N-terminal additional sequence of an expressed protein (e.g., Factor Xa sequence). Examples of such polypeptides include, but are not limited to, a polypeptide having an RNase III activity that has the amino acid sequence of SEQ ID NO:5. Furthermore, the composition of the present invention may contain a buffer for stabilizing the polypeptide.

The composition may contain a protein having an activity of binding to a nucleic acid. A cold shock protein can be preferably used as the protein having an activity of binding to a nucleic acid although it is not intended to limit the present invention. For example, a cold shock protein (Csp) derived from a thermophilic bacterium or a thermostable bacterium can be preferably used. A CspB protein derived from *Thermotoga maritima* having the amino acid sequence of SEQ ID NO:12 (an amino acid sequence encoded by the nucleotide sequence of SEQ ID NO:13) is preferable.

In another embodiment of the composition of the present invention, the composition may contain a fusion protein of the above-mentioned protein having an activity of binding to a nucleic acid and the polypeptide having an RNase III activity of the present invention.

### (4) Kit used for the method of the present invention

The kit used for the method of the present invention is a kit for efficiently conducting a reaction of degradation into a dsRNA of a specific length. For example, it is a kit for efficiently preparing an siRNA of a length with which an effect in RNA interference can be expected.
The kit of the present invention may contain a polypeptide having an RNase III activity. One as described in (1) above can be preferably used as the polypeptide having an RNase III activity. Furthermore, the kit of the present invention may contain a buffer for stabilizing the polypeptide.

The kit may contain a protein having an activity of binding to a nucleic acid. A cold shock protein can be preferably used as the protein having an activity of binding to a nucleic acid although it is not intended to limit the present invention. For example, a cold shock protein (Csp) derived from a thermophilic bacterium or a thermostable bacterium can be preferably used. A CspB protein derived from *Thermotoga maritima* having the amino acid sequence of SEQ ID NO:12 (an amino acid sequence encoded by the nucleotide sequence of SEQ ID NO:13) is preferable.

In another embodiment of the kit of the present invention, the kit may contain a fusion protein of the above-mentioned protein having an activity of binding to a nucleic acid and the polypeptide having an RNase III activity of the present invention.

The kit of the present invention may contain a component other than those as described above such as a reagent for synthesizing a dsRNA as a substrate, a reagent for purifying a dsRNA of a specific length produced as a result of the reaction, or a reagent for transferring it into a biological sample. Although it is not intended to limit the present invention, for example, it may contain a reagent for purifying an siRNA as a degradation product, a regent for transferring it into a biological sample or the like.
It is possible to conveniently and efficiently prepare an siRNA of a length with which an effect in RNA interference can be expected using the kit of the present invention.

### Examples

The following Examples illustrate the present invention in more detail, but are not to be construed to limit the scope thereof.

### Example 1: Preparation of shotgun library

### (1) Preparation of genomic DNA

*Shewanella* sp. Ac10 was inoculated into 5 ml of a medium containing 0.15 g/ml Tryptone, 0.01 g/ml yeast extract, 0.3 g/ml sodium chloride, 0.01 g/ml glucose, 11.5 mM dipotassium hydrogenphosphate, 7.4 mM potassium dihydrogenphosphate and 4 mM magnesium sulfate, and cultured at 15°C for 40 hours. The culture was centrifuged at 5000 rpm for 1 minute. The cells obtained as a precipitate were suspended in 500 µl of a solution containing 25 mM Tris-HCl buffer (pH 8.0), 50 mM glucose and 10 mM EDTA. 50 µl of 10% SDS was added to the suspension and the mixture was mixed adequately. 5 µl of 20 mg/ml Protease K (Takara Bio) was added thereto. The mixture was incubated at 50°C for three hours. 500 µl of chloroform was further added thereto. The mixture was gently shaken for 30 minutes and centrifuged at 10000 x g for 10 minutes to obtain a supernatant. 1.5 ml of ethanol was added to the supernatant. A genomic DNA in a cotton-like form was collected by winding using a tip, washed twice with 70% ethanol, dried at 50°C and dissolved in 100 µl of a solution containing 1 mM Tris-HCl buffer (pH 7.5) and 0.1 mM EDTA to obtain a genomic DNA solution.

### (2) Construction of library

The concentration of the genomic DNA solution obtained in Example 1-(1) was adjusted to 2 µg/200 µl. The genomic DNA was cleaved to result in average chain lengths of 1.0 kbp using Hydrosher (Gene Machines) under conditions of cycle 20 and speed 4. 2 µl of Pellet paint (Novagen), 20 µl of 3 M sodium acetate (pH 5.2) and 600 µl of ethanol were added to the solution of cleaved DNA for ethanol precipitation. The precipitate was dried and dissolved in 20 µl of TE buffer (10 mM Tris-HCl buffer (pH 7.5) and 1 mM EDTA). 5 ul of the solution was subjected to a blunting reaction, a kination reaction and a ligation reaction using TaKaRa BKL Kit (Takara Bio) according to the attached protocol. pUC18 SmaI/BAP (Amersham Pharmacia) or pUC118 HincII/BAP (Takara Bio) was used as a vector for the ligation reaction. The volume of the solution obtained using the kit was adjusted to 100 µl with TE buffer. It was subjected to phenol/chloroform extraction and ethanol precipitation, and the precipitate was dissolved in 20 µl of TE buffer. 50 µl of DH10B electro cell (Gibco BRL) as *Escherichia coli* competent cells was added to the ligation mixture, and electroporation was carried out using Gene Pulser (Bio-Rad) under conditions of 1.5 kV, 200 Q and 25 µF. 1 ml of SOC medium was added to the solution, the mixture was shaken at 37°C for one hour, and cultivation was carried out on LB agar medium containing 100 µg/ml ampicillin, 100 µg/ml IPTG and 40 µg/ml X-gal. As a result, about 1000 colonies appeared. Inserts were checked by PCR reactions for a part of colonies. Thus, a shotgun library consisting of 4 million clones was prepared.

### Example 2: Analysis of genomic sequence

### (1) Determination of genomic sequence

Plasmid DNAs were prepared from 43,200 *Escherichia coli* clones in the shotgun library obtained in Example 1 as follows. An *Escherichia coli* clone was cultured at 37°C for 14 hours in 200 µl of a medium containing 1 g/ml Tryptone, 0.5 g/ml yeast extract and 1 g/ml sodium chloride. The culture was centrifuged at 5000 rpm for 1 minute. The cells obtained as a precipitate were suspended in 80 µl of a solution containing 25 mM Tris-HCl buffer (pH 8.0), 50 mM glucose and 10 mM EDTA using a plasmid extraction robot (Beckman). 80 µl of a solution containing 0.2 N sodium hydroxide and 1% SDS was added thereto, and 80 µl of 3 M potassium acetate solution (pH 5.2) was further added. The suspension was transferred to NA plate (Millipore), and cell debris was removed by aspiration. The clarified solution was transferred to FB plate (Millipore) to which 150 µl of 8 M guanidine solution had been added. The plasmid DNA was adsorbed to the FB plate by aspiration. After washing three times with 80% ethanol, the plasmid DNA was eluted with 70 µl of a solution containing 10 mM Tris-HCl buffer (pH 8.0) and 1 mM EDTA.

0.875 µl of sterile water, 0.125 µl each of primers M13-47 and RV-M (10 pmol/µl, both from Takara Bio) and 2 µl of DynamicET were added to 2 µl of the plasmid DNA. The mixture was subjected to a PCR as follows: 40 cycles of 95°C for 30 seconds, 50°C for 20 seconds and 60°C for 1 minute and 20 seconds. The reaction mixture was subjected to nucleotide sequence analysis using a sequencer MegaBACE 1000 (Amersham Pharmacia). The thus obtained nucleotide sequence data were subjected to assembly, alignment and analysis using a computer program CAP4 (Pracel) to construct a single genomic sequence.

### (2) Analysis of gene

ORF analyses were conducted for the genomic sequence obtained in Example 2-(1) using a computer program Genmark v.2.4c (Gene Probe, Inc.). As a result, 4935 ORFs were deduced. Homology searches were conducted for the ORFs using a computer algorithm BLAST (Ver 2.0) against a gene database GenBank. Then, functions were estimated for 3057 of the ORFs. Information about homologies of the respective ORFs to enzymes was obtained by the BLAST. searches. A gene of interest from the cold-adapted microorganism *Shewanella* sp. Ac10 that was presumed to encode an RNase III and has the nucleotide sequence of SEQ ID NO:1 was obtained from them.

### Example 3: Cloning and expression of RNase III

### (1) Construction of expression vector

An expression vector was constructed as follows.
First, synthetic primers 1 and 2 having nucleotide sequences of SEQ ID NOS:2 and 3, respectively, were synthesized using a DNA synthesizer based on the nucleotide sequence for the RNase III obtained in Example 2-(2) and purified according to a conventional method. The synthetic primer 1 is a synthetic DNA that has a recognition sequence for a restriction enzyme EcoRI at nucleotides 11-16 and a nucleotide sequence corresponding to amino acids 1-7 in the amino acid sequence of the RNase III (SEQ ID NO:4) at nucleotides 18-37. The synthetic primer 2 has a recognition sequence for a restriction enzyme BamHI at nucleotides 11-16 and a nucleotide sequence corresponding to amino acids 221-226 in the amino acid sequence of the RNase III (SEQ ID NO:4) at nucleotides 20-37.

A PCR was carried out using the above-mentioned synthetic primers. Reaction conditions for the PCR were as follows.
Briefly, a reaction mixture of a total volume of 100 µl was prepared by adding 1 µl of the genomic DNA prepared in Example 1-(1), 10 µl of 10 x Pyrobest buffer (Takara Bio), 8 µl of dNTP Mixture (Takara Bio), 20 pmol each of the synthetic primers 1 and 2, 5 U of Pyrobest DNA polymerase (Takara Bio) and sterile water. The reaction mixture was placed in TaKaRa PCR Thermal Cycler MP (Takara Bio) and subjected to a reaction as follows: 30 cycles of 94°C for 30 seconds, 55°C for 30 seconds and 72°C for 2 minutes.

After reaction, 5 µl of the reaction mixture was subjected to electrophoresis on 1.0% agarose gel. The observed about 680-bp DNA fragment of interest was recovered and purified from the electrophoretic gel and subjected to ethanol precipitation. After ethanol precipitation, the recovered DNA was suspended in 64 µl of sterile water, and doubly digested with restriction enzymes EcoRI (Takara Bio) and BamHI (Takara Bio). The EcoRI-BamHI digest was extracted and purified after electrophoresis on 1.0% agarose gel to obtain an EcoRI-BamHI-digested DNA fragment.

Next, *Escherichia coli* JM109 transformed with a plasmid vector pMM047 was cultured. This strain was designated and indicated as *Escherichia coli* JM109/pMM047 and deposited at International Patent Organism Depositary, National Institute of Advanced Science and Technology (AIST Tsukuba Central 6, 1-1, Higashi 1-Chome, Tsukuba, Ibaraki 305-0566, Japan) under accession number FERM P-16496 on October 31, 1997 (date of original deposit), and deposited at International Patent Organism Depositary, National Institute of Advanced Science and Technology under accession number FERM BP-6523 (date of request for transmission to international depositary authority: September 24, 1998). The plasmid vector pMM047 was purified according to a conventional method. A vector pCold08NC2 was prepared based on this vector according to the method as described in Examples 1-6 in WO 99/27117.

The vector pCold08 was cleaved with the same restriction enzymes as those used for the preparation of the EcoRI-BamHI-digested DNA fragment, and the termini were dephosphorylated. The vector was then mixed and ligated with the EcoRI-BamHI-digested DNA fragment using DNA Ligation Kit Ver. 1 (Takara Bio). 20 µl of the ligation mixture was used to transform *Escherichia coli* JM109, and transformants were grown on LB medium (containing 50 µg/ml of ampicillin) containing agar at a concentration of 1.5%(w/v).

A plasmid into which the DNA fragment of interest was inserted was confirmed by sequencing. This recombinant plasmid was designated as pCold08 SH-RNIII. This plasmid was designated and indicated as pCold08 SHE-RNIII and deposited at International Patent Organism Depositary, National Institute of Advanced Science and Technology (AIST Tsukuba Central 6, 1-1, Higashi 1-Chome, Tsukuba, Ibaraki 305-8566, Japan) under accession number FERM P-19526 on September 22, 2003, and deposited at International Patent Organism Depositary, National Institute of Advanced Science and Technology under accession number FERM BP-10075 (date of request for transmission to international depositary authority: July 28, 2004).

### (2) Expression and purification

pCold08 SHE-RNIII prepared in Example 3-(1) was used to transform *Escherichia coli* BL21. Transformants were grown on LB medium (containing 50 µg/ml of ampicillin) containing agar at a concentration of 1.5%(w/v). A grown colony was inoculated into 2.5 ml of LB liquid medium (containing 50 µg/ml of ampicillin) and cultured at 37°C overnight. A portion of the culture was inoculated into 100 ml of LB medium and cultured at 37°C until the logarithmic growth phase. After cultivation, the culture was shaken for 10 minutes in an incubator at 15°C, IPTG was added thereto at a final concentration of 1.0 mM, and the cultivation was continued at 15°C for 24 hours for expression induction. The cells were collected by centrifugation, and resuspended in 5 ml of a cell disruption solution (50 mM tris-hydrochloride buffer (pH 8.5), 100 mM sodium chloride, 0.5 mM EDTA, 1% Triton X-100, 1 mM dithiothreitol, 2 mM phenylmethylsulfonyl fluoride). The cells disrupted by sonication were subjected to centrifugation (11,000 rpm, 20 minutes) to separate a supernatant extract from a precipitate.

About 5 ml of the supernatant extract was further purified using a nickel column as follows.
10 ml of buffer A (20 mM tris-hydrochloride buffer (pH 8.5), 100 mM sodium chloride, 1 mM dithiothreitol, 0.1% Triton X-100) was added to and mixed with Ni-NTA agarose (Qiagen) corresponding to a resin volume of 1 ml. The mixture was centrifuged at 1,500 rpm for several minutes. The supernatant was discarded and about 1 ml of the resin was collected. About 5 ml of the supernatant prepared from the disrupted cells was added to the resin, and mixed gently using a rotary shaker at 4°C for about one hour. Then, the resin to which the protein of interest had been adsorbed was filled in a ϕ 15-mm column, and washed twice with 5 ml of buffer A. The resin was then washed with 5 ml of buffer B (20 mM tris-hydrochloride buffer (pH 8.5), 100 mM sodium chloride, 1 mM dithiothreitol, 0.1% Triton X-100, 40 mM imidazole). The resin was further washed with 5 ml of buffer C (20 mM tris-hydrochloride buffer (pH 8.5), 800 mM sodium chloride, 1 mM dithiothreitol, 0.1% Triton X-100, 40 mM imidazole) followed by 5 ml of buffer B to remove unnecessary proteins other than the protein of interest.

After washing, elution was carried out with 3 ml of buffer D (20 mM tris-hydrochloride buffer (pH 8.5), 100 mM sodium chloride, 1 mM dithiothreitol, 0.1% Triton X-100, 100 mM imidazole). The eluate was dialyzed against 500 ml of buffer E (50 mM tris-hydrochloride buffer (pH 8.5), 100 mM sodium chloride, 0.5 mM EDTA, 0.1% Triton X-100, 1 mM dithiothreitol) and concentrated about 10-fold (about 300 µl) using Centricon (Amicon). When a portion of the purified and concentrated sample was subjected to electrophoresis on 10% SDS-polyacrylamide gel, a band for the protein of interest was observed at a position corresponding to a molecular weight of about 27,500. The protein concentration was about 4 mg/ml. When Western blotting detection with an anti-His tag antibody was carried out for the sample using Anti His HRP Conjugate (Qiagen) according to the attached protocol, the band for the protein of interest was detected upon color development.
The protein is a polypeptide having an RNase III activity that has amino acid sequences including a Perfect DB sequence, a His tag sequence and a Factor Xa sequence being added, and the amino acid sequence of SEQ ID NO:5.

### Example 4: Measurement of RNase III activity

### (1) Activity of degrading dsRNA

An activity of degrading a dsRNA of the RNase III sample prepared in Example 3-(2) was measured. The activity was measured as follows.
A dsRNA as a substrate used for the activity measurements was synthesized using TurboScript T7 Transcription kit (GTS) according to the attached protocol.
Specifically, pDON-rsGFP was constructed by inserting a gene encoding red-shift green fluorescent protein (hereinafter referred to as rsGFP) (SEQ ID NO:6) from a plasmid pQBI125 (Wako Pure Chemical Industries) into a plasmid pDON-AI (Takara Bio). A PCR was carried out using pDON-rsGFP as a template as well as a primer dsr-1 (SEQ ID NO:7) which has a T7 promoter sequence and a primer dsr-2 (SEQ ID NO:8) to obtain an amplification product. An about 700-bp dsRNA was prepared by an RNA synthesis reaction using the resulting double-stranded DNA as a template and T7 RNA polymerase (hereinafter referred to as rsGFP-dsRNA).

A reaction mixture of a total volume of 10 µl was prepared by adding 5 µg of rsGFP-dsRNA prepared as described above, 1 µl of the protein sample prepared in Example 3-(2), 1 µl of 30 mM magnesium chloride solution, 2 µl of 5 x reaction buffer (250 mM tris-hydrochloride buffer (pH 8.5), 1.25 M sodium chloride, 0.5% Triton X-100, 5 mM dithiothreitol) and nuclease-free water.
In case of a commercially available RNase III from *Escherichia coli* (Epicentre) as a control, a reaction mixture of a total volume of 10 µl was prepared by adding 1 µl of the enzyme solution and 5 µg of rsGFP-dsRNA as a substrate to a buffer containing 33 mM tris-acetate (pH 7.8), 66 mM potassium acetate, 10 mM magnesium acetate and 0.5 mM dithiothreitol.

After the above-mentioned reaction mixtures were prepared and reacted at 30°C (in case of the polypeptide having an RNase III activity from the cold-adapted microorganism) or 37°C (in case of the RNase III from *Escherichia coli*) for a given period of time, 5 µl each of the reaction mixtures were subjected to electrophoresis on 15% polyacrylamide gel and staining with ethidium bromide to observe cleavage products.

As a result, using the polypeptide having an RNase III activity from the cold-adapted microorganism, cleavage products of 100 base pairs or longer, which are indicative of the partial degradation stage, were observed after cleavage for 30 minutes. Cleaved fragments larger than 20 base pairs were observed even after cleavage for one hour. A cleavage pattern almost the same as that of the cleavage for one hour was observed after cleavage overnight (about 18 hours). The main degradation product was observed at a position corresponding to a length of about 20 base pairs. On the other hand, in case of the RNase III from *Escherichia coli,* it was confirmed that the main product after cleavage for 30 minutes was a small degradation product of about 10 base pairs.
Based on the above, it was confirmed that the polypeptide having an RNase III activity from the cold-adapted microorganism of the present invention does not tend to produce a small degradation product of about 10 base pairs, and that one can readily prepare a degradation product by partial degradation.

### (2) Examination of temperature sensitivity

The temperature sensitivity of the activity of degrading a dsRNA was examined using the RNase III sample prepared in Example 3-(2). An RNase III from *Escherichia coli* (Epicentre) was used as a control. The activity was measured as follows.
An enzyme solution of a total volume of 5 µl was prepared by adding 1 µl of the protein sample prepared in Example 3-(2), 1 µl of 30 mM magnesium chloride solution, 2 µl of 5 x reaction buffer (250 mM tris-hydrochloride buffer (pH 8.5), 1.25 M sodium chloride, 0.5% Triton X-100, 5 mM dithiothreitol) and nuclease-free water. It was treated at 30°C, 40°C, 50°C or 60°C for 10 minutes or 20 minutes. 5 µg of rsGFP-dsRNA used in Example 4-(1) was added thereto. The resulting mixture of a total volume of 10 µl was reacted at 30°C for one hour.

In case of the RNase III from *Escherichia coli* (Epicentre) as a control, an reaction mixture of a total volume of 5 µl was prepared by adding 1 µl of the enzyme solution to a buffer containing 33 mM tris-acetate (pH 7.8), 66 mM potassium acetate, 10 mM magnesium acetate and 0.5 mM dithiothreitol. It was treated at 40°C, 50°C, 60°C or 70°C for 10 minutes or 20 minutes. 5 µg of rsGFP-dsRNA was added thereto. The resulting mixture of a total volume of 10 µl was reacted at 37°C for one hour.
After reaction, 5 µl each of the reaction mixtures was subjected to electrophoresis on 15% polyacrylamide gel and staining with ethidium bromide to observe cleavage products.

As a result, an activity of degrading a dsRNA was not observed after treatment at 50°C for 20 minutes in case of the polypeptide having an RNase III activity from the cold-adapted microorganism. On the other hand, the activity was not observed after treatment at 60°C for 20 minutes in case of the RNase III from *Escherichia coli.*
Thus, it was shown that the polypeptide having an RNase III activity from the cold-adapted microorganism is more temperature-sensitive and can be inactivated at a lower temperature than the RNase III from *Escherichia coli.*

### Example 5

The RNA interference effect of a dsRNA degradation product prepared using the polypeptide having an RNase III activity from the cold-adapted microorganism of the present invention was examined. A commercially available RNase III from *Escherichia coli* was used as a control. dsRNA degradation products were prepared according to the method as described in Example 4-(1). Specifically, dsRNA degradation products were prepared by cleaving 15 µg of rsGFP-dsRNA at 30°C for 60 minutes (in case of the RNase III from the cold-adapted microorganism), at 37°C for 60 minutes (in case of complete degradation using the RNase III from *Escherichia coli)* or at 37°C for 10 minutes (in case of partial degradation using the RNase III from *Escherichia coli).* The cleavage products were purified using RNA Purification Column 1, 2 (Gene Therapy Systems) and used for assessments as follows.

Assessments in RNA interference were carried out as follows.
Briefly, an appropriate number (5 x 10⁴ cells) of 293 cells were seeded into a 24-well plate containing D-MEM medium (Sigma) supplemented with 10% FBS and 1% penicillin/streptomycin 24 hours before transfer of a dsRNA degradation product, and cultured overnight in a CO₂ incubator. The cells were cultured to about 80% confluence. 3 µl of TransIT 293 Transfection Reagent (Takara Bio) was added to 50 µl of a serum-free medium. The mixture was vigorously stirred and allowed to stand at room temperature for 5 minutes. 0.3 µg of pQBI25 (Wako Pure Chemical Industries) was added thereto, and the mixture was gently mixed and allowed to stand at room temperature for 5 minutes. 4 µl of TransIT-TKO reagent was added thereto, and the mixture was gently mixed and allowed to stand at room temperature for 5 minutes. 500 ng of the dsRNA degradation product prepared above was added thereto, and the mixture was gently mixed and allowed to stand at room temperature for 5 minutes to obtain a DNA/dsRNA degradation product solution. The DNA/dsRNA degradation solution was added dropwise to 250 µl of a serum-free medium in each well, and the mixture was mixed gently so that the solution in the well became homogeneous. 0.3 µg of pQBI25 or sterile water was added alone to controls. The cells were cultured for 24 hours in a CO₂ incubator. The cells were subjected to flow cytometry using FACS Vantage (Becton-Dickinson) to determine the inhibitory effect of the transferred DNA/dsRNA degradation product solution on rsGFP expression as compared with the control with the vector (DNA) alone. The results are shown in Table 1.

**Table 1**

| Transferred sample | Average fluorescence intensity |
|---|---|
| Control (no addition) | 8.09 |
| Control (vector alone) | 1331.44 |
| *E. coli* RNase III (complete degradation) | 1035.36 |
| *E. coli* RNase III (partial degradation) | 637.30 |
| *Shewanella* sp. Ac10 RNase III | 295.14 |

A less average fluorescence value as compared with the control (vector alone) as shown in Table 1 represents more RNA interference. It was confirmed that the degradation product with the RNase III from *Shewanella* sp. Ac10 exhibited an RNA interference effect stronger than the complete or partial degradation product with the RNase III from *Escherichia coli.*
Based on the above, it was confirmed that the polypeptide having an RNase III activity from the cold-adapted microorganism of the present invention is useful for preparation of an siRNA for RNA interference.

### Example 6: RNase III activity on another dsRNA substrate

dsRNA degradation activities of the RNase III samples prepared in Example 3-(2) were assessed using, as a substrate, a dsRNA prepared from a luciferase gene. A dsRNA was synthesized using TurboScript T7 Transcription kit (GTS) according to the attached protocol in a manner similar to that in Example 4-(1).

Specifically, PCRs were carried out to obtain two amplification products from the luciferase-encoding gene inserted into the plasmid pGL3-Basic vector (Promega) using the plasmid pGL3-Basic vector (Promega) as a template as well as a primer dsl-1 (SEQ ID NO:9) which has a T7 promoter sequence and a primer dsl-2 (SEQ ID NO:10) (the length of amplified fragment: about 500 base pairs), or the primer dsl-1 and a primer dsl-3 (SEQ ID NO:11) (the length of amplified fragment: about 1000 base pairs). A dsRNA of about 500 bp or about 1000 bp was prepared by an RNA synthesis reaction using one of the resulting double-stranded DNAs as a template and T7 RNA polymerase.

A reaction mixture of a total volume of 10 µl was prepared by adding 5 µg of the dsRNA prepare as described above, 1 µl of the protein sample prepared in Example 3-(2), 1 µl of 30 mM magnesium chloride solution, 2 µl of 5 x reaction buffer (250 mM tris-hydrochloride buffer (pH 8.5), 1.25 M sodium chloride, 0.5% Triton X-100, 5 mM dithiothreitol) and nuclease-free water.
After the above-mentioned reaction mixture was prepared and reacted at 30°C for a given period of time, 5 µl of the reaction mixture was subjected to electrophoresis on 15% polyacrylamide gel and staining with ethidium bromide to observe cleavage products.

As a result, cleaved fragments larger than 20 base pairs were observed even after cleavage for one hour as cleavage products. A cleavage pattern almost the same as that of the cleavage for one hour was observed after cleavage overnight (about 18 hours).
Based on the above, it was confirmed that the polypeptide having an RNase III activity from the cold-adapted microorganism of the present invention does not tend to produce a small degradation product of about 10 base pairs, and that one can readily prepare a degradation product by partial degradation even if the template is changed or the length of the dsRNA as a substrate is changed.

### Example 7: Examination of factors that contribute to production and degradation of dsRNA

(1) A protein having an activity of binding to a nucleic acid in a normal temperature range was examined in order to examine factors that contribute to production and degradation of a dsRNA.
   It was difficult to obtain such a protein having an activity of binding to a nucleic acid. Then, a CspB protein from *Thermotoga maritima* having the amino acid sequence of SEQ ID NO:12 was used as a model protein. The protein was prepared according to the method as described in Protein Science, 8:394-403 (1999).

(2) Effect of CspB from *Thermotoga maritima* on dsRNA degradation
An activity of degrading a dsRNA with the addition of CspB was measured as follows.
Briefly, when the RNase III was used as an enzyme, a reaction mixture of a total volume of 10 µl was prepared by adding 1 µl of the protein sample (enzyme solution) prepared in Example 3-(2), 1 µl of a CspB solution prepared in (1) above, 1 µg of a dsRNA as a substrate, 1 µl of 30 mM magnesium chloride solution, 2 µl of 5 x reaction buffer (250 mM tris-hydrochloride buffer (pH 8.5), 1.25 M sodium chloride, 0.5% Triton X-100, 5 mM dithiothreitol) and nuclease-free water.
The CspB protein was added at a final concentration of 9.2 ng/µl, 18.4 ng/µl or 92 ng/µl. 1 µl of 10 mM potassium phosphate buffer (pH 7.5) as a shape buffer for CspB was added to a control (no addition).
After the above-mentioned reaction mixtures were prepared and reacted at 30°C for one hour, 5 µl each of the reaction mixtures was subjected to electrophoresis on 15% polyacrylamide gel and staining with ethidium bromide to observe cleavage products.

As a result, promotion of dsRNA degradation due to the addition of CspB was observed at each amount.

### Example 8: Assessment of dsRNA degradation sample prepared from rsGFP-dsRNA

The RNA interference effect of a dsRNA degradation product prepared using the RNase III from the cold-adapted microorganism of the present invention was examined. A commercially available *E. coli* RNase III (Epicentre) was used as a control. A dsRNA degradation product was prepared basically according to the method as described in Example 4-(1). Specifically, 10 µg of rsGFP-dsRNA prepared in Example 4-(1) was cleaved at 30°C for one hour using 2 µl of the RNase III from *Shewanella* as described in Example 3-(2). In case of the commercially available *E. coli* RNase III (1 U/µl), 10 µg of the dsRNA was cleaved at 37°C for 10 minutes (partial degradation) or 60 minutes (complete degradation) using 2 µl of the RNase III. The cleavage products were purified using RNA Purification Column 1, 2 (Gene Therapy Systems) and used for assessments in RNA interference as follows.

Briefly, an appropriate number (1.5 x 10⁵ cells) of 293 cells were seeded into a 24-well plate containing D-MEM medium (Sigma) supplemented with 10% FBS and 1% penicillin/streptomycin 24 hours before transfer of an siRNA, and cultured overnight in a CO₂ incubator. The cells were cultured to about 80% confluence. 1 µl of Genejuice Transfection Reagent (Takara Bio) was added to 49 µl of a serum-free medium. The mixture was vigorously stirred and allowed to stand at room temperature for 5 minutes. 0.3 µg of pQBI25 (Wako Pure Chemical Industries) was added thereto, and the mixture was gently mixed and allowed to stand at room temperature for 5 minutes.
In parallel, 3 µl of Ribojuice Transfection Reagent (Takara Bio) was added to 47 µl of a serum-free medium in another tube, and the mixture was vigorously stirred. After allowing to stand at room temperature for 5 minutes, 55.6 ng of the dsRNA degradation product was added thereto, and the mixture was gently mixed and allowed to stand at room temperature for 5 minutes.
One of the two types of solutions prepared as described above was added dropwise to 250 µl of D-MEM medium supplemented with 10% FBS in each well, and the mixture was mixed gently so that the solution in the well became homogeneous. The vector (DNA) or sterile water was added alone to controls. The cells were cultured for 24 hours in a CO₂ incubator. The cells were subjected to flow cytometry using FACS Vantage (Becton-Dickinson) to determine the inhibitory effect of the transferred DNA/dsRNA degradation product solution on rsGFP expression as compared with the control with the vector (DNA) alone. The results are shown in Table 2.

**Table 2**

| Transferred sample | Average fluorescence intensity (relative value) |
|---|---|
| Control (no addition) | 0 |
| Control (vector alone) | 100 |
| *Shewanella* sp.AC10 RNase III | 49.19 |
| Commercially available *E. coli* RNase III (partial degradation) | 77.62 |
| Commercially available *E. coli* RNase III (complete degradation) | 93.81 |

A less average fluorescence intensity value as compared with the control (vector alone) as shown in Table 2 represents more RNA interference. It was confirmed that the dsRNA degradation product obtained using the RNase III from *Shewanella* sp. AC10 exhibited an RNAi effect like the one obtained using the commercially available *E. coli* RNase III, and the exhibited RNA interference effect was stronger than that of the one obtained using the commercially available *E. coli* RNase III.
Based on the above, it was confirmed that the RNase III of the present invention is useful for preparation for RNA interference.

Total RNAs extracted from the cell samples prepared in Example 6 were subjected to real-time RT-PCR to quantify the mRNAs for rsGFP for assessment in RNA interference.

Specifically, a total RNA was extracted and purified from cells cultured for 24 hours at 37°C in a CO₂ incubator after transfer of an siRNA using trizole (Invitrogen) according to the attached protocol. A reaction mixture of a total volume of 20 µl was prepared by adding 80 ng of the total RNA, 4 µl of 5 x M-MLV buffer (Takara Bio), 1 µl of 10 mM dNTPs (Takara Bio), 100 pmol of random hexamer, 20 U of RNase Inhibitor (Takara Bio), 100 U of M-MLV Reverse Transcriptase and sterile water. The reaction mixture was placed in TaKaRa PCR Thermal Cycler SP (Takara Bio) and subjected to a reaction at 42°C for 10 minutes and then at 95°C for 2 minutes. 20 µl of a reaction dilution solution (1 x M-MLV buffer, 0.5 mM dNTP mixture) was added to the reaction mixture. 10 µl aliquots of the resulting mixture were dispensed. 2.5 µl of 10 x R-PCR buffer (Takara Bio), 0.3 µl of 250 mM Mg²⁺, 0.75 µl of 10 mM dNTPs, 1.25 U of TaKaRa Ex Taq R-PCR (Takara Bio), 2.5 µl of a 3000-fold dilution of SYBR Green (Takara Bio) in sterile water and 1.25 µl of 100% DMSO were added to 10 µl of the reaction mixture. 5 pmol each of synthetic primers for detecting β-actin (Takara Bio), GAPDH (Takara Bio), rsGFP (rsGFP-F (SEQ ID NO:14), rsGFP-R (SEQ ID NO:15)) or Neo (Neo-F (SEQ ID NO:16), Neo-R (SEQ ID NO:17)) and sterile water to a total volume of 25 µl were further added. The reaction mixtures were placed in Smart Cycler II Unit (Takara Bio), heat-denatured at 95°C for 10 seconds, and subjected to a reaction as follows: 45 cycles of 95°C for 5 seconds and 60°C for 20 seconds. The obtained data were analyzed to quantify the mRNAs for human β-actin and GAPDH as well as rsGFP and Neo from the transferred plasmid. The results are shown in Table 3.

**Table 3**

| Transferred sample | Amount of rsGFP mRNA (relative value) |
|---|---|
| Control (no addition) | 0 |
| Control (vector alone) | 100 |
| *Shewanella* sp.AC10 RNase III | 36.85 |
| Commercially available *E*. *coli* RNase III (partial degradation) | 51.36 |
| Commercially available *E. coli* RNase III (complete degradation) | 72.30 |

It is considered that a less amount of rsGFP mRNA as compared with the control (vector alone) as shown in Table 3 represents more RNA interference. It was confirmed that the dsRNA degradation product obtained using the RNase III from *Shewanella* sp. AC10 exhibited an effect like the one obtained using the commercially available *E. coli* RNase III, and the exhibited RNA interference effect was stronger than that of the one obtained using the commercially available *E. coli* RNase III.
Based on the above, it was confirmed that the RNase III of the present invention is useful for preparation of an siRNA for RNA interference.

### Example 9: Assessment of dsRNA degradation product prepared from dsRNA from luciferase

The procedure as described in Example 8 was carried out using the sample prepared in Example 3-(2) and a dsRNA from luciferase as a substrate. The RNA interference effect of the dsRNA degradation product was examined. The 500-bp one prepared in Example 6 was used as the dsRNA from luciferase.

dsRNA degradation products were prepared basically according to the method as described in Example 4-(1). Specifically, 10 µg of the dsRNA was cleaved at 30°C for one hour using 2 µl of the *Shewanella* RNase III in Example 3-(2), or at 37°C for 10 minutes (partial degradation) or 60 minutes (complete degradation) using 2 µl of the commercially available *E. coli* RNase III (1 U/µl). The cleavage products were purified using RNA Purification Column 1, 2 (Gene Therapy Systems) and used for assessments in RNA interference as follows. The product of cleavage at 37°C for 10 minutes with the *E. coli* RNase III was subjected to polyacrylamide gel electrophoresis, and a band corresponding to a length of about 21 bp was excised. TE buffer was added to the excised gel piece, the mixture was shaken at room temperature for 16 hours, and the eluate was subjected to ethanol precipitation to recover the cleavage product which was used as a gel-recovery sample.

Assessments in RNA interference were carried out as follows. Briefly, an appropriate number (1.5 x 10⁵ cells) of 293 cells were seeded into a 24-well plate containing D-MEM medium (Sigma) supplemented with 10% FBS and 1% penicillin/streptomycin 24 hours before transfer of a dsRNA degradation product, and cultured overnight in a CO₂ incubator. The cells were cultured to about 80% confluence. 1 µl of Genejuice Transfection Reagent (Takara Bio) was added to 49 µl of a serum-free medium. The mixture was vigorously stirred and allowed to stand at room temperature for 5 minutes. 0.5 µg of pGL3-control (Promega) and 0.1 µg of pRL-TK (Promega) were added thereto, and the mixture was gently mixed and allowed to stand at room temperature for 5 minutes.
In parallel, 3 µl of Ribojuice Transfection Reagent (Takara Bio) was added to 47 µl of a serum-free medium in another tube, and the mixture was vigorously stirred. After allowing to stand at room temperature for 5 minutes, 500 ng, 166.7 ng or 55.6 ng of the siRNA was added thereto, and the mixture was gently mixed and allowed to stand at room temperature for 5 minutes.
One of the two types of solutions prepared as described above was added dropwise to 250 µl of D-MEM medium supplemented with 10% FBS in each well, and the mixture was mixed gently so that the solution in the well became homogeneous. The vector (DNA) or sterile water was added alone to controls. The cells were cultured for 24 hours in a CO₂ incubator. The cells were subjected to an assay using Dual Luciferase Reporter assay kit (Promega) according to the attached instructions to determine the inhibitory effect of the addition of the dsRNA degradation product on GL3 protein expression as compared with the control with the vector (DNA) alone. The results are shown in Table 4.

**Table 4**

| Transferred siRNA sample | GL3 expression level (relative value) |
|---|---|
| Control (no addition) | 0 |
| Control (vector alone) | 100 |
| *Shewanella* sp.AC10 RNase III 500 ng | 10.71 |
| *Shewanella* sp.AC10 RNase III 166.7 ng | 11.33 |
| *Shewanella* sp.AC10 RNase III 55.6 ng | 19.06 |
| Commercially available *E. coli* RNase III (partial degradation) 500 ng | 10.13 |
| Commercially available *E. coli* RNase III (partial degradation) 166.7 ng | 13.43 |
| Commercially available *E. coli* RNase III (partial degradation) 55.6 ng | 29.83 |
| Commercially available *E. coli* RNase III (complete degradation) 500 ng | 19.60 |
| Commercially available *E. coli* RNase III (complete degradation) 166.7 ng | 44.84 |
| Commercially available *E. coli* RNase III (complete degradation) 55.6 ng | 72.56 |
| Commercially available *E. coli* RNase III (gel-recovery) 500 ng | 8.73 |
| Commercially available *E. coli* RNase III (gel-recovery) 166.7 ng | 16.58 |
| Commercially available *E. coli* RNase III (gel-recovery) 55.6 ng | 39.69 |

It is considered that a less GL3 expression level as compared with the control (vector alone) as shown in Table 4 represents more RNA interference. It was confirmed that the dsRNA degradation product obtained using the *Shewanella* RNase III exhibited an RNA interference effect like the one obtained using the commercially available E. *coli* RNase III, and the exhibited RNA interference effect was stronger than that of the one obtained using the commercially available *E. coli* RNase III. It was further shown that the effect was superior to that of the gel-recovered cleavage product.
Based on the above, it was confirmed that the RNase III of the present invention is useful for preparation of an siRNA for RNA interference.

### Example 10: Comparison between Shewanella RNase III and Dicer from human

The RNA interference effect of a dsRNA prepared using the *Shewanella* RNase III was compared with the RNA interference effect of a dsRNA prepared using a Dicer from human. The assessment system using luciferase as described in Example 9 was utilized.

A commercially available Dicer (GTS) was used as a Dicer from human. The practical procedure followed the method as described in Example 9 except that the 293 cells to be subjected to the transfer of the dsRNA degradation product were cultured to 95% confluence in a CO₂ incubator. The results are shown in Table 5.

**Table 5**

| Transferred siRNA sample | GL3 mRNA amount (relative value) |
|---|---|
| Control (no addition) | 0 |
| Control (vector alone) | 100 |
| *Shewanella* RNase III 500 ng | 9.42 |
| *Shewanella* RNase III 166.7 ng | 10.50 |
| *Shewanella* RNase III 55.6 ng | 21.22 |
| *Shewanella* RNase III 18.5 ng | 42.33 |
| Commercially available Dicer from human 166.7 ng | 8.21 |
| Commercially available Dicer from human 55.6 ng | 9.73 |
| Commercially available Dicer from human 18.5 ng | 16.85 |

It is considered that a less GL3 mRNA amount as compared with the control (vector alone) as shown in Table 5 represents more RNA interference. It was confirmed that the siRNA obtained using the *Shewanella* RNase III exhibited an RNA interference effect equivalent to the one obtained using the commercially available Dicer.
Based on the above, it was confirmed that the RNase III of the present invention is useful for preparation of an siRNA for RNA interference.

### Industrial Applicability

The present invention provides a polypeptide having an RNase III activity with which a length of a dsRNA degradation product can be readily controlled by reaction conditions. The polypeptide can be used to prepare an siRNA optimal for RNA interference. Furthermore, a method for preparing an siRNA which is useful for RNA interference and the like is provided according to the method of the present invention. In addition, the present invention provides a composition and a kit with which the method of the present invention can be conveniently carried out.

### Sequence Listing Free Text

SEQ ID NO:2; Synthetic primer 1 to amplify a gene encoding Shewanella sp.AC10 RNaseIII
SEQ ID NO:3; Synthetic primer 2 to amplify a gene encoding Shewanella sp.AC10 RNaseIII
SEQ ID NO:5; An expression peptide sequence of Shewanella sp.AC10 RNaseIII '
SEQ ID NO:7; Synthetic primer dsr-1 to amplify a gene encoding red-shifted green fluorescence protein
SEQ ID NO:8; Synthetic primer dsr-2 to amplify a gene encoding red-shifted green fluorescence protein
SEQ ID NO:9; Synthetic primer dsl-1 to amplify a gene encoding luciferase
SEQ ID NO:10; Synthetic primer dsl-2 to amplify a gene encoding luciferase
SEQ ID NO:11; Synthetic primer dsl-3 to amplify a gene encoding luciferase
SEQ ID NO:14; Synthetic primer rsGFP-F to amplify a gene encoding rsGFP
SEQ ID NO:15; Synthetic primer rsGFP-R to amplify a gene encoding rsGFP
SEQ ID NO:16; Synthetic primer Neo-F to amplify a gene encoding Neo
SEQ ID NO:17; Synthetic primer Neo-R to amplify a gene encoding Neo

## Claims

1. A polypeptide having an RNase III activity, which is derived from a microorganism, and with which a dsRNA degradation product of a length within a specific range that is effective for RNA interference can be obtained after complete degradation.

2. A polypeptide having an RNase III activity, for which reaction conditions can be readily controlled, and with which a dsRNA degradation product of a length within a specific range larger than a final degradation product obtained by treating a dsRNA with an RNase III from *Escherichia coli* can be obtained.

3. A polypeptide having an RNase III activity, of which the dsRNA degradation velocity is slower than the dsRNA degradation velocity of an RNase III from *Escherichia coli,* and for which reaction conditions can be readily controlled.

4. A polypeptide having an RNase III activity, of which the dsRNA degradation velocity is slower than the dsRNA degradation velocity of an RNase III from *Escherichia coli*, for which reaction conditions can be readily controlled, and which does not tend to produce a small dsRNA degradation product of about 10 base pairs.

5. The polypeptide according to any one of claims 1 to 4, which is derived from a cold-adapted microorganism.

6. The polypeptide according to claim 5, wherein the cold-adapted microorganism is a microorganism of the genus *Shewanella.*

7. A polypeptide having an RNase III activity, with which a dsRNA degradation product of a length within a specific range larger than a final degradation product obtained by treating a dsRNA with an RNase III from *Escherichia coli* can be obtained, and which contains an amino acid sequence selected from the group consisting of:
(a) the amino acid sequence of SEQ ID NO:4;
(b) an amino acid sequence in which one or several amino acid(s) is(are) substituted, deleted, inserted or added in the amino acid sequence of SEQ ID NO:4; and
(c) an amino acid sequence encoded by a nucleotide sequence that is capable of hybridizing to the nucleotide sequence of SEQ ID NO:1 under stringent conditions.

8. The polypeptide according to any one of claims 1 to 7, which is a fusion protein with a protein having an activity of binding to a nucleic acid.

9. A method for degrading a dsRNA, the method comprising allowing the polypeptide defined by any one of claims 1 to 8 to act on a dsRNA.

10. The method according to claim 9, wherein the dsRNA degradation product is a dsRNA that is capable of functioning in RNA interference as an siRNA.

11. The method according to claim 9 or 10, which is conducted in the presence of a protein having an activity of binding to a nucleic acid.

12. The method according to claim 11, wherein the protein having an activity of binding to a nucleic acid is a cold shock protein derived from a thermophilic bacterium or a thermostable bacterium.

13. The method according to claim 12, wherein the cold shock protein is cold shock protein B from *Thermotoga maritima.*

14. A composition for degrading a dsRNA, which is used for the method defined by any one of claims 9 to 13, and which contains the polypeptide having an RNase III activity defined by any one of claims 1 to 8.

15. A kit for degrading a dsRNA, which is used for the method defined by any one of claims 9 to 13, and which contains the polypeptide having an RNase III activity defined by any one of claims 1 to 8.

16. A nucleic acid that encodes a polypeptide having an RNase III activity, which has a nucleotide sequence selected from the group consisting of:
(a) the nucleotide sequence of SEQ ID NO:1;
(b) a nucleotide sequence in which one or several nucleotide(s) is(are) substituted, deleted, inserted or added in the nucleotide sequence of SEQ ID NO:1; and
(c) a nucleotide sequence that is capable of hybridizing to the nucleotide sequence of SEQ ID NO:1 under stringent conditions.

17. A method for producing a polypeptide having an RNase III activity, the method comprising culturing a host cell containing the nucleic acid defined by claim 16, and collecting a polypeptide having an RNase III activity from the culture.
